# EUROPEAN PATENT APPLICATION

(11) **EP 4 818 322 A1**
(43) Date of publication of application: **07.10.2026**
(21) Application number: 24895314.3
(22) Date of filing: 06.02.2024
(51) Int. Cl.: C12N 15/47, C07K 14/145, A61K 47/12, A61P 31/14, C07K 16/10, A61K 39/42

(54) **RABIES VACCINE IMMUNOGEN COMPOSITION**

(30) Priority: 29.11.2023 CN 202311609502
(71) Applicant: Zhongshan Hospital, Fudan University, Shanghai 200032 (CN)
(72) Inventor: XU, Jianqing, Shanghai 200032 (CN); ZHANG, Xiaoyan, Shanghai 200032 (CN); BAI, Shimeng, Shanghai 200032 (CN)
(74) Representative: Bayramoglu et al.
(86) International application number: PCT/CN2024/076204
(87) International publication number: WO 2025/112200

(57) **Abstract**

An anti-rabies virus immunogenic composition capable of simultaneously inducing a humoral immune response and a cellular immune response, and use thereof. The anti-rabies virus immunogenic composition relates to a dual immunogenic combination including rabies virus glycoprotein G as an immunogen for activating neutralizing antibodies and polymerase large protein L as a T-cell immunogen. The rabies virus glycoprotein G is derived from full-length glycoprotein, extracellular domain amino acids thereof, or an antigenic fragment thereof; the T-cell immunogen is derived from a recombinant sequence of a conserved region of the polymerase large protein. The two immunogens can be optionally loaded into the same type of vector or different types of vectors. The vaccine is safe, can simultaneously activate anti-rabies virus T-cell responses and antibody responses to achieve combined protection, and is used for the prevention and treatment of rabies virus infection.

## Description

### TECHNICAL FIELD

The present invention belongs to the field of biomedicine and relates to genetic engineering vaccine technology. Specifically, it relates to a dual-antigen mRNA rabies vaccine capable of simultaneously inducing a humoral immune response and a cellular immune response, its construction, and its application in the prevention and treatment of rabies.

### BACKGROUND TECHNOLOGY

Rabies is a zoonotic disease with an almost 100% mortality rate after clinical symptoms appear, making it one of the deadliest known infectious diseases. Rabies virus replicates rapidly and can paralyze the host's central nervous system within a few days, but it can be prevented by early and timely pre- or post-exposure vaccination. Therefore, the vaccine used must immediately induce a strong immune response, including both a humoral immune response to produce sufficient rabies virus-neutralizing antibodies to neutralize the virus before it reaches the central nervous system, and an induced cellular immune response to eliminate virus-infected cells. Although the clearance of rabies virus depends on antibodies, T cell-driven mechanisms contribute to controlling viral replication, particularly mediated by IFN-γ produced by infiltrating T cells. In the central nervous system, both an innate immune response capable of inhibiting viral replication and active, infiltrating rabies virus-specific T cells and B cells that can completely eliminate the virus are required. It has been previously reported that rabies vaccination, whether pre- or post-exposure, stimulates a cellular immune response. Specifically, the cytokines IFN-γ, IL-2, and TNF-α secreted by Th1-type cells are important for antiviral immunity, as they promote the interaction between CD8+ T cells and dendritic cells and help B cells produce high-affinity neutralizing antibodies. The cytokines secreted by Th2-type cells (such as IL-4, IL-5, and IL-13) also play a key role in B cell-mediated humoral immune responses.

In recent years, with the maturation of mRNA vaccine technology, rabies mRNA vaccines developed by many countries have entered preclinical or clinical trial stages. For example, CV7202 developed by CureVac AG of Germany has entered Phase I clinical trials; LVRNA001 from Zhuhai Lifanda Biotechnology Co., Ltd. has been submitted for clinical trial application; and the rabies mRNA vaccine of Everest Medicines has completed conceptual validation. All these vaccines are designed to use rabies virus glycoprotein RABV-G as the immunogen to induce neutralizing antibodies and exert protective immune responses.

### CONTENT OF THE INVENTION

Currently, no vaccine manufacturers have explored the targeted induction of T-cell immune responses, particularly the elimination of virus-infected cells mediated by cytotoxic T lymphocytes.

Driven by the research goal of enhancing immune protection through dual immunity, our team is committed to developing a rabies vaccine that can rapidly induce both humoral immune responses and robust cellular immune responses. This vaccine can rapidly inhibit viral replication at infection sites to prevent and control the spread of rabies virus, especially when administered at an early stage or post-exposure, enabling faster host immune activation. Accordingly, under the premise of ensuring efficacy, the design of a potent vaccine targeting both humoral and cellular immunity must enable efficient processing and presentation of B-cell and T-cell immunogen epitopes. This maximizes the induction of neutralizing antibodies and activates T cells to exert immune protection. Simple fusion expression of B-cell and T-cell immunogens fails to achieve optimal efficacy. Therefore, the induction of specific cellular immune responses must not significantly compromise the humoral immune response that generates neutralizing antibodies.

In short, the technical problem solved by the present invention is to provide a dual immunogenic composition that can rapidly induce both humoral and cellular immune responses. The B-cell immunogen for inducing humoral immunity is derived from rabies virus glycoprotein (G), and the T-cell immunogen for activating cellular immunity is derived from the polymerase large protein (L). Both immunogens are delivered using the same vector, such that their respective immune responses do not interfere with each other, thereby achieving effective prevention and/or treatment of rabies.

To solve this technical problem, the present invention provides a dual immunogenic composition capable of simple, convenient, and efficient co-expression of two exogenous genes. When used in vaccine preparation, the vaccine exhibits favorable immunogenicity, can rapidly establish potent immune protection and induce strong cellular immune responses after immunization, and features low production cost and simple preparation procedures. It is suitable for rapid, efficient, and large-scale production of various vaccines, and holds great development potential.

Specifically, the present invention includes the following technical solutions:

Provided is an immunogenic composition including: rabies virus structural protein glycoprotein (G) as an immunogen for activating neutralizing antibodies; and polymerase large protein L as a T-cell immunogen for inducing cellular immune responses.

The immunogens are derived from widely circulating rabies virus strains of different genotypes, including but not limited to Pasteur strains such as Pitman-Moore (PM) strain, Pasteur (PV) strain, CTN strain, aG strain, Flury-LEP strain, Evelyn-Rokitnicki-Abelseth (ERA) strain, Street-Alabama-Duffering (SAD) strain, and related viruses: KHUV (Khujand lyssavirus), BBLV (Bokeloh bat lyssavirus), ARAV (Aravan lyssavirus), EBLV-1 (European bat 1 lyssavirus), EBLV-2 (European bat 2 lyssavirus), IRKV (Irkut lyssavirus), LBV (Lagos bat lyssavirus), SHIBV (Shimoni bat lyssavirus), MOKV (Mokola lyssavirus), WCBV (West Caucasian bat lyssavirus), IKOV (Ikoma lyssavirus), DUVV (Duvenhage lyssavirus), ABLV (Australian bat lyssavirus), GBLV (Gannoruwa bat lyssavirus), and LLEBV (Lleida bat lyssavirus).

The method for obtaining the T-cell antigen sequence includes the following steps:
i. Performing sequence similarity alignment on the amino acid sequences of multiple L proteins (Large Protein) of rabies virus;
ii. Predicting T-cell epitopes based on the obtained similarity sequence information to acquire information on immunogenic conserved regions. This includes predicting polypeptides in the L protein that may strongly bind to HLA supertype molecules, i.e., polypeptides that may be presented, by using online tools;
iii. Designing and preparing different immunogenic sequences or polypeptide sequences according to the conserved region information. This includes mapping these polypeptides to the corresponding target L protein, further comprehensively analyzing enriched regions among different vaccine strains, and truncating the enriched regions to form a recombinant immunogen amino acid sequence of the L protein, as shown in SEQ ID NO: 5;
iv. Combining the obtained immunogenic sequence or coding sequence with other sequences for co-expression (e.g., fusion expression or separate expression), such as antigen sequences capable of inducing a humoral immune response.

Based on the above research, the technical solutions provided by the present invention include the following:
An immunogenic composition for a rabies vaccine, where the immunogenic composition is a protein-form immunogenic composition or a DNA-form immunogenic composition, and the DNA is a gene encoding the protein, where
the protein-form immunogenic composition includes:
   A. rabies virus structural protein glycoprotein G (abbreviated as "rabies virus G protein"), as a humoral immunogen for activating neutralizing antibodies (abbreviated as "antibody immunogen"), for inducing a humoral immune response; and
   B. polymerase large protein L, as a T-cell immunogen for inducing a cellular immune response (abbreviated as "cellular immunogen"), for inducing a cellular immune response;
the DNA-form immunogenic composition includes:
   a. a nucleic acid molecule a encoding the rabies virus structural protein glycoprotein G of item A; and
   b. a nucleic acid molecule b encoding the polymerase large protein L of item B.

In the immunogenic composition for a rabies vaccine, the rabies virus structural protein glycoprotein G is a full-length amino acid sequence of glycoprotein G, an extracellular domain amino acid sequence of glycoprotein G, or an antigenic fragment of glycoprotein G, etc.

The full-length amino acid sequence of glycoprotein G is selected from the group consisting of:
(1) a polypeptide having the amino acid sequence as shown in SEQ ID NO: 1; and
(2) a polypeptide having at least 80%, at least 85%, at least 90%, at least 91%, at least 92%, at least 93%, at least 94%, at least 95%, at least 96%, at least 97%, at least 98%, or at least 99% homology with the amino acid sequence as shown in SEQ ID NO: 1 and having the same function as SEQ ID NO: 1, the function referring to the function of inducing a humoral immune response and producing neutralizing antibodies;

The extracellular domain amino acid sequence of glycoprotein G is selected from the group consisting of:
(3) a polypeptide having the amino acid sequence as shown in SEQ ID NO: 2; and
(4) a polypeptide having at least 80%, at least 85%, at least 90%, at least 91%, at least 92%, at least 93%, at least 94%, at least 95%, at least 96%, at least 97%, at least 98%, or at least 99% homology with the amino acid sequence as shown in SEQ ID NO: 2 and having the same function as SEQ ID NO: 1, the function referring to the function of inducing a humoral immune response and producing neutralizing antibodies; and
the polymerase large protein L is selected from the group consisting of:
(5) a polypeptide having the amino acid sequence as shown in SEQ ID NO: 5; and
(6) a polypeptide having at least 80%, at least 85%, at least 90%, at least 91%, at least 92%, at least 93%, at least 94%, at least 95%, at least 96%, at least 97%, at least 98%, or at least 99% homology with the amino acid sequence as shown in SEQ ID NO: 5 and having the same function as SEQ ID NO: 5, the function referring to the function of inducing a cellular immune response.

Preferably, when the nucleic acid molecule a encodes the polypeptide of SEQ ID NO: 1, the nucleic acid molecule a is selected from the group consisting of:
(7) the nucleotide sequence as shown in SEQ ID NO: 3; and
(8) a nucleotide sequence having at least 80%, at least 85%, at least 90%, at least 91%, at least 92%, at least 93%, at least 94%, at least 95%, at least 96%, at least 97%, at least 98%, or at least 99% homology with SEQ ID NO: 3;
when the nucleic acid molecule a encodes the polypeptide of SEQ ID NO: 2, the nucleic acid molecule a is selected from the group consisting of:
(9) the nucleotide sequence as shown in SEQ ID NO: 4; and
(10) a nucleotide sequence having at least 80%, at least 85%, at least 90%, at least 91%, at least 92%, at least 93%, at least 94%, at least 95%, at least 96%, at least 97%, at least 98%, or at least 99% homology with SEQ ID NO: 4;
when the nucleic acid molecule b encodes the polypeptide of SEQ ID NO: 5, the nucleic acid molecule b is selected from the group consisting of:
(11) the nucleotide sequence as shown in SEQ ID NO: 6, the nucleotide sequence is a sequence optimized for codons and RNA secondary structure; and
(12) a nucleotide sequence having at least 80%, at least 85%, at least 90%, at least 91%, at least 92%, at least 93%, at least 94%, at least 95%, at least 96%, at least 97%, at least 98%, or at least 99% homology with SEQ ID NO: 6.

A second aspect of the present invention resides in the use of the immunogenic composition for a rabies vaccine in the preparation of a rabies vaccine.

Preferably, the rabies vaccine can induce both humoral immune responses and cellular immune responses.

In a preferred embodiment of the use, the nucleic acid molecule a and nucleic acid molecule b may be inserted into an expression vector to construct a vector vaccine. The vector used may be selected from the group consisting of: nucleic acid vaccine vectors, namely mRNA vectors and DNA plasmid vectors; recombinant viral vectors; and recombinant bacterial vectors.

Further, the mRNA vectors may be selected from linear, circular, and self-replicating vectors; and the recombinant viral vectors are selected from vectors such as vaccinia virus, adenovirus, adeno-associated virus, herpes simplex virus, measles virus, reovirus, rhabdovirus, tick-borne encephalitis virus, influenza virus, respiratory syncytial virus, and poliovirus.

In one embodiment, the nucleic acid molecule a and nucleic acid molecule b may be inserted into vectors of the same type or different types. Insertion into multiple vectors of the same type results in a multivalent vaccine; insertion into different vectors results in a combined vaccine. It will be readily understood by those skilled in the art that both multivalent vaccines and combined vaccines can activate both anti-rabies virus T cell responses and antibody responses through sequential or combined immunization, thereby achieving combined protection.

The recombinant adenovirus vector may be selected from the group consisting of adenovirus type 5, adenovirus type 11, adenovirus type 26, adenovirus type 35, adenovirus type 63, adenovirus type 68, and the like.

The recombinant vaccinia virus vector may be selected from the group consisting of Tiantan strain, North American vaccine strain, Wyeth-derived strain, Lister strain, Ankara-derived strain, Copenhagen strain, New York strain, and the like, and is preferably the Tiantan strain.

In certain aspects of the present invention, the nucleic acid molecule a (antibody immunogen) and nucleic acid molecule b (T-cell immunogen) may be inserted into an mRNA expression vector including a 5'UTR, a 3'UTR, a poly(A) sequence, and a coding sequence, wherein the coding sequence includes different signal peptide sequences, different degradation domains, internal ribosome entry site sequences derived from different sources (non-coding region mRNA sequences in viruses and intracellular mRNA sequences), and an antigen-coding region (including a first protein domain and a second protein domain).

In some embodiments, the expression vector further includes, from the 5' end to the 3' end, essential elements required for transcription, specifically a T7 promoter region, a 5' untranslated region, a coding sequence, a 3' non-coding region, and a poly(A) tail sequence; all four essential elements must be included. The UTR sequence, the 3'-UTR sequence, and the poly(A) tail sequence are the sequences in the optimal combination as described in Chinese Patent Application No. 202211129466.2.

In some embodiments, the coding sequence includes a signal peptide sequence, an antigen-coding region, and different degradation domains.

The signal peptide sequence includes a native signal peptide sequence of a transmembrane antigen and a signal peptide sequence of a secreted antigen. The secreted signal peptide sequence may be a signal peptide sequence of mouse IL-2, a light-chain signal peptide sequence of an IgG antibody, a heavy-chain signal peptide sequence of an IgG antibody, a human erythropoietin signal peptide sequence, a bovine prolactin signal peptide sequence, a tissue plasminogen signal peptide sequence, or an influenza hemagglutinin signal peptide sequence. According to a specific embodiment of the present invention, a native signal peptide sequence derived from a transmembrane antigen is preferred.

The antigen-coding region is a pathogen antigen-coding sequence including, in the N-terminal to C-terminal direction, a first protein domain, an internal ribosome entry site sequence, and a second protein domain. The first protein domain and the second protein domain may be antigens of various infectious disease pathogens. The pathogen antigens include antigens capable of specifically inducing humoral immune responses and T-cell antigens capable of activating cellular immune responses mediated by cytotoxic T lymphocytes.

In the antigen-coding region, different protein tags are inserted into the pathogen antigen-coding sequence, which may be at least one of a Myc tag, an HA tag, a Flag tag, or a His tag. According to a specific embodiment of the present invention, a Flag tag sequence is preferred.

Further, the first protein domain may be an antigen sequence that specifically induces a humoral immune response, and the second protein domain may be a T-cell antigen sequence capable of activating a cellular immune response; or the first protein domain may be a T-cell antigen sequence capable of activating a cellular immune response, and the second protein domain may be an antigen sequence that specifically induces a humoral immune response. According to a specific embodiment of the present invention, it is further preferred that the first protein domain is an antigen sequence inducing a humoral immune response, and the second protein domain is a T-cell antigen sequence activating a cellular immune response.

The antigen-coding region sequence is bicistronic or multicistronic, and the coding sequence in the bicistronic or multicistronic mRNA encodes an antigen sequence of various infectious disease pathogens as defined in the present invention, or a fragment or variant thereof.

Further, the bicistronic or multicistronic mRNA is separated by at least one IRES (internal ribosome entry site) sequence, thereby recruiting ribosomes to achieve mutually independent translation of the pathogen antigen sequences or fragments or variants thereof.

The internal ribosome entry site (IRES) sequence includes non-coding region mRNA sequences derived from viruses and intracellular mRNA sequences.

Further, the viral IRES sequence is preferably a non-coding region mRNA sequence derived from a virus, including different types of viruses such as DNA viruses and RNA viruses. Specifically, DNA viruses may be herpesviruses (e.g., Kaposi's sarcoma-associated herpesvirus), polyomaviruses (e.g., simian vacuolating virus); RNA viruses may be picornaviruses (e.g., poliovirus, coxsackievirus, enterovirus, hepatitis A virus, foot-and-mouth disease virus, encephalomyocarditis virus, classical swine fever virus), hepatitis C virus, classical swine fever virus, retroviruses (e.g., murine leukemia virus, human T-cell leukemia virus type 1, Rous sarcoma virus, simian immunodeficiency virus, human immunodeficiency virus). According to a specific embodiment of the present invention, a sequence derived from encephalomyocarditis virus as shown in SEQ ID NO: 7 is further preferred.

The different degradation domains are selected from Salmonella virulence factor SopE, estrogen receptor domain mutant 2 (ERmut2), FKB protein (FKBP, FK506-binding protein) domain, or dihydrofolate reductase (DHFR). According to a specific embodiment of the present invention, an estrogen receptor (ER) domain mutant 2-derived domain as shown in SEQ ID NO: 8 is further preferred; a dihydrofolate reductase (DHFR)-derived domain as shown in SEQ ID NO: 9 is even further preferred.

Further, the degradation domain is located at the N-terminus of the T-cell antigen sequence capable of activating cellular immune responses (either the first protein domain or the second protein domain), or at the C-terminus of the T-cell antigen sequence capable of activating cellular immune responses. According to a specific embodiment of the present invention, location at the C-terminus of the T-cell antigen sequence capable of activating cellular immune responses is further preferred.

In some embodiments, the first protein domain is RABV-G, an antigen sequence that induces humoral immune responses, which is highly conserved among various rabies virus strains. For example, RABV-G may be derived from the Pitman-Moore (PM) strain, whose amino acid sequence is shown in SEQ ID NO: 1.

In certain aspects of the present invention, the second protein domain is a T-cell antigen sequence that induces cellular immune responses. Specifically, early-expressed viral proteins (such as various proteins involved in the viral replication cycle) are selected as the main immunogens; sequences from various strains of different pathogens are collected, consensus sequences are extracted therefrom, and T-cell epitope prediction is performed, followed by use of conserved regions with high immunogenicity, either alone or in combination.

Further, the cytotoxic T lymphocyte epitopes include a plurality of different consensus sequences, mainly linked in tandem, and a flexible linker or a rigid linker may be added. Cytotoxic T lymphocyte epitopes can be predicted by bioinformatics methods such as artificial neural networks (ANN). Prediction software is known in the art and includes, but is not limited to, tools available at http://www.bio.med.ucm.es/episopt.html, http://www.ddg-pharmfac.net/mhcpred/MHCPred, http://www.syfpeithi.de/software, and the like.

The third aspect of the present invention provides a rabies vaccine capable of simultaneously inducing a humoral immune response and a cellular immune response. The vaccine is obtained by the above-mentioned use, and is a nucleic acid vaccine, an adenovirus vector vaccine, or an inactivated cell vaccine.

In a preferred embodiment, the rabies vaccine is an mRNA vaccine, which is constructed by inserting the nucleic acid molecule a and nucleic acid molecule b into an mRNA vector. The inserted mRNA expression vector includes, in order from 5' end to 3' end, a T7 promoter region, a 5' untranslated region, a coding sequence, a 3' non-coding region, a poly(A) tail sequence, and a single restriction enzyme site. The coding sequence is an immunogen coding region including, in the N-terminal to C-terminal direction, a first protein sequence, an internal ribosome entry site sequence, a second protein sequence, and a degradation domain. The first protein sequence is one of a rabies virus structural protein glycoprotein G coding sequence and a polymerase large protein L coding sequence, and the second protein sequence is the other one of the rabies virus structural protein glycoprotein G coding sequence and the polymerase large protein L coding sequence.

In the immunogen coding region, the first protein sequence is the antigen sequence that induces neutralizing antibodies to generate a humoral immune response, namely the rabies virus structural protein glycoprotein G coding sequence (abbreviated as the first protein domain G protein). The second protein sequence is the T-cell antigen sequence that activates a cellular immune response, namely the polymerase large protein L coding sequence (abbreviated as the second protein domain recombinant L protein).

The internal ribosome entry site (IRES) sequence may be the nucleotide sequence derived from encephalomyocarditis virus as shown in SEQ ID NO: 7.

The degradation domain may be selected from the estrogen receptor (ER) domain, FKB protein (FKBP) domain, or dihydrofolate reductase (DHFR). Preferably, the degradation domain is the dihydrofolate reductase (DHFR) nucleotide sequence as shown in SEQ ID NO: 9, or a nucleotide sequence having at least 90%, 95%, 97%, 98%, 99%, or 100% homology with SEQ ID NO: 9.

Preferably, the rabies vaccine is a dual-antigen rabies mRNA vaccine. The sequence included in the mRNA expression vector is in the following order from the 5' end to the 3' end: a T7 promoter, a UTR sequence, a first protein domain G protein, an IRES sequence, a second protein domain recombinant L protein, a degradation domain, a 3'-UTR sequence, a poly(A) tail sequence, and a single restriction enzyme site, and the like.

In some aspects of the present invention, there is also provided use of the immunogenic polypeptide sequence of the present invention, namely the T-cell antigen sequence RABV-LT, in resisting rabies virus infection.

In some embodiments, the T-cell antigen sequence can be used alone against rabies virus infection, and can be further used in T-cell vaccines or drugs for anti-rabies virus infection. In some embodiments, the use includes the full sequence of the T-cell immunogenic peptide of the present invention, or a partial sequence thereof, or a conjugate thereof.

In some aspects of the present invention, some or all of the uracil and/or cytosine nucleosides are replaced during the transcription of RABV-G, RABV-LT and the dual-antigen RABV-G-LT to prepare mRNA.

Further, the rabies vaccine is a dual-antigen rabies mRNA vaccine and is a chemically modified mRNA vaccine. The modification includes replacing at least 50%, at least 60%, at least 70%, at least 80%, at least 90%, or 100% of the uracil in the mRNA vaccine backbone with a substance selected from the group consisting of: uridine, N1-methylpseudouridine, N1-ethylpseudouridine, 2-thiouridine, 4'-thiouridine, 5-methylcytosine, 5-methyluridine, 2-thio-1-methyl-1-deazapseudouridine, 2-thio-T-methylpseudouridine, 2-thio-5-azauridine, 2-thiodihydropseudouridine, 2-thiodihydrouridine, 2-thiopseudouridine, 4-methoxy-2-thiopseudouridine, 4-methoxypseudouridine, 4-thio-1-methylpseudouridine, 4-thiopseudouridine, 5-azauridine, dihydropseudouridine, 5-methoxyuridine, and 2'-O-methyluridine.

It is well known to those skilled in the art that simple chemical modification of the mRNA backbone is crucial for the success of mRNA vaccines. Such modifications enable mRNA vaccines to induce strong immune responses without causing significant side effects and also improve the stability of mRNA. For example, the COVID-19 mRNA vaccines developed by Moderna and Pfizer/BioNTech employ chemically modified mRNA. Numerous studies have demonstrated that modified mRNA represents the best-performing technology for prophylactic vaccines.

The above substance is preferably pseudouridine, N1-methylpseudouridine, or N1-ethylpseudouridine, and more preferably N1-methylpseudouridine.

Preferably, the rabies vaccine further includes, in addition to the immunogen main body, one or more pharmaceutically or veterinarily acceptable carriers or excipients. The carriers or excipients are preferably carriers or excipients that render the rabies vaccine suitable for oral, intradermal, subcutaneous, intramuscular, or intranasal administration.

In some aspects of the present invention, there is also provided a formulation including an mRNA vaccine prepared from a sequence that induces a humoral immune response (such as RABV-G), an mRNA vaccine prepared from a T-cell antigen sequence RABV-LT that induces a cellular immune response, and/or an mRNA vaccine prepared from a dual-antigen sequence RABV-G-LT that induces both humoral and cellular immune responses. The formulation also includes a delivery vehicle for the above mRNA vaccines for prophylaxis and/or treatment. The vehicle includes cationic liposomes, polymers, proteins, or lipid nanoparticles, preferably cationic liposomes and cationic lipid nanoparticles, and more preferably cationic lipid nanoparticles. The formulation can be prepared by mixing the cationic lipid nanoparticles with the mRNA vaccine.

It is readily understood that the rabies vaccine is used for vaccination of humans or animals to prevent rabies virus infection, or for therapeutic vaccination of humans after being bitten or scratched by animals to prevent the onset of rabies, especially in combination with anti-rabies virus serum or antibodies.

The present invention provides an mRNA vector capable of efficiently expressing multiple different types of antigen molecules simultaneously. The mRNA vector encodes a bicistronic sequence loaded with internal ribosome entry site sequences from different sources, enabling simultaneous targeting of humoral and cellular immune responses. Different degradation domains are also added to the antigen-coding region, so that the T-cell immunogen targeting cellular immune response is rapidly transported to the proteasome for degradation after translation and expression, thereby better presenting T-cell epitopes and activating cytotoxic T lymphocytes to exert immune protection. Moreover, the humoral and cellular immune responses do not significantly interfere with each other when different antigen molecules in the antigen-coding region stimulate the body. In addition, the mRNA vector can be used to prepare the dual-antigen rabies mRNA vaccine RABV-G-LT, enabling rapid induction of effective immune responses after vaccination for the prevention and treatment of rabies virus infection.

### DESCRIPTION OF THE DRAWINGS

The present disclosure is further described below with reference to the drawings. These drawings are only for illustrating the embodiments of the present disclosure and are not intended to limit the scope of the present disclosure.

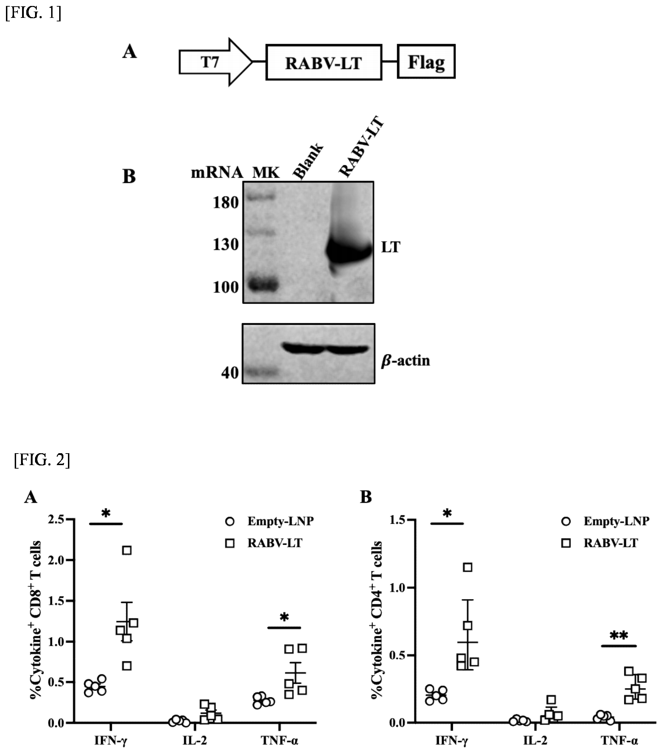

FIG. 1 shows the construction and verification of the recombinant T-cell antigen RABV-LT.
FIG. 2 shows the immunogenicity evaluation of RABV-LT mRNA vaccine in BALB/c mice. The mice used in the experiment were female BALB/c mice aged 6-8 weeks, and the immunogen was RABV-LT mRNA vaccine. It includes antigen-specific CD8+ T-cell response (A) and antigen-specific CD4+ T-cell response (B) induced by RABV-LT mRNA vaccine at 7 days after a single dose. ns: not significant; *: p<0.05; **: p<0.01; ***: p<0.001; ****: p<0.0001.

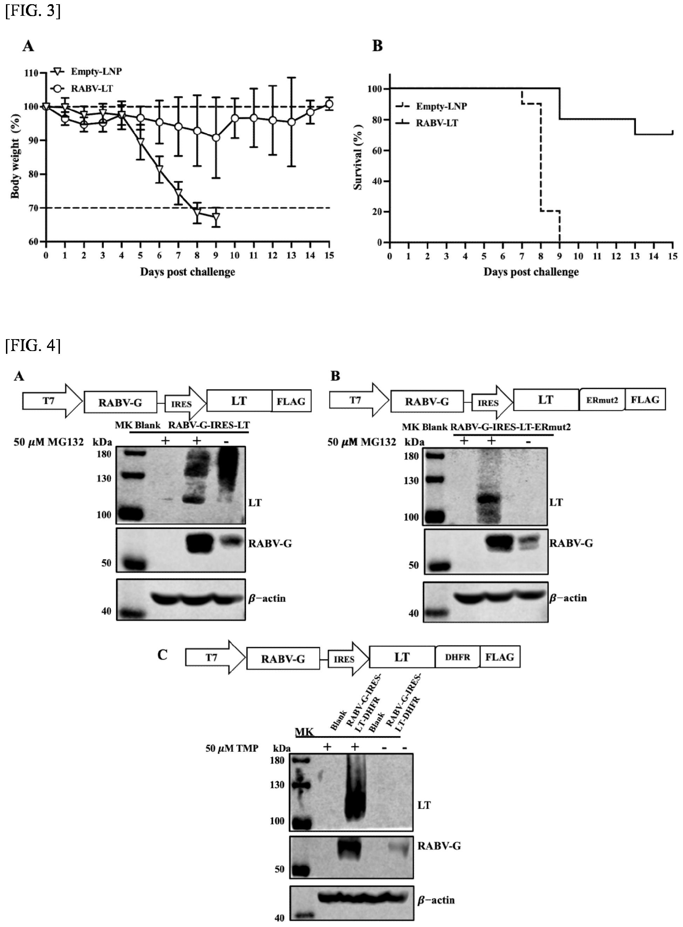

FIG. 3 shows the protective efficacy of RABV-LT mRNA vaccine against viral challenge, including comparison of body weight changes (A) and survival rates (B) of mice in different groups after challenge.
FIG. 4 shows the construction and verification of a dual-antigen RABV-G-LT molecule incorporating RABV-LT antigen and RABV-G antigen. It includes RABV-G-IRES-LT antigen (A), RABV-G-IRES-LT-ERmut2 antigen conjugated with estrogen receptor domain mutant 2 (ERmut2) (B), and RABV-G-IRES-LT-DHFR antigen conjugated with dihydrofolate reductase (DHFR) (C), respectively.

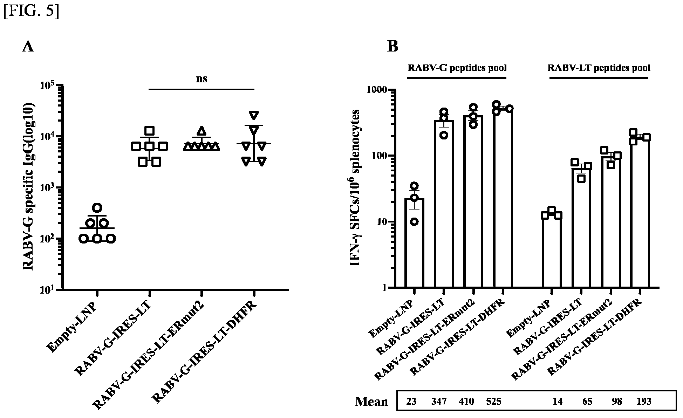

FIG. 5 shows the immunogenicity evaluation of different dual-antigen RABV-G-LT mRNA vaccines in BALB/c mice. It includes detection of RABV-G-specific binding antibody titers in mouse serum at 12 days after immunization by ELISA (A), and detection of T-cell response in mouse spleens at 12 days after immunization by ELISPOT (B). The abscissa represents the immunization groups, and the ordinate represents the number of IFN-γ-secreting cells per million splenocytes. ns or unmarked: not significant; *: p<0.05; **: p<0.01; ***: p<0.001; ****: p<0.0001.

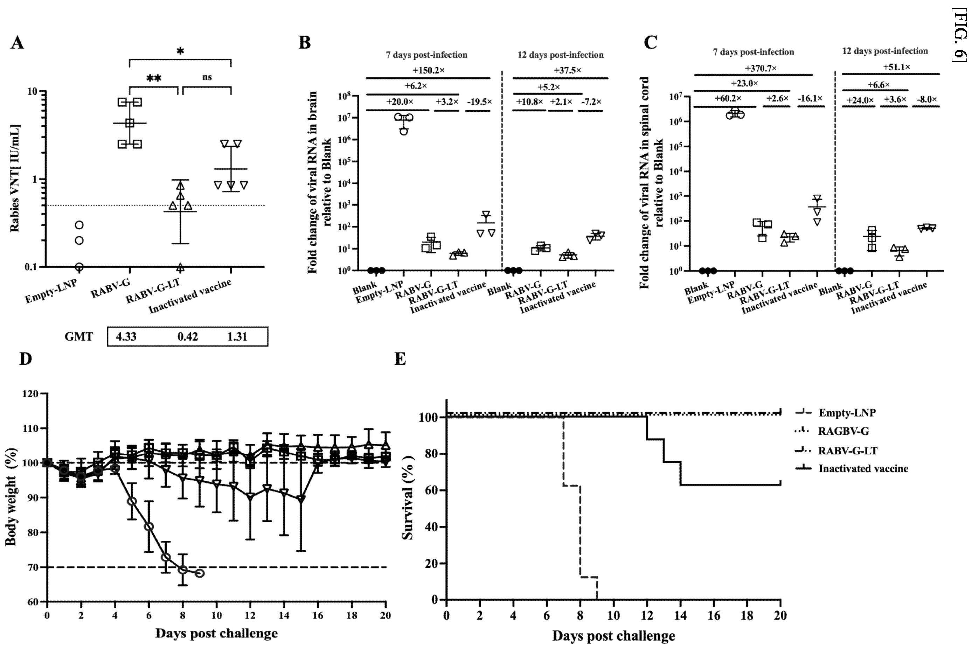

FIG. 6 shows the protective efficacy evaluation of RABV-G-LT mRNA vaccine, RABV-G mRNA vaccine, and commercial inactivated vaccine against lethal rabies virus challenge at 7 days after vaccination. It includes live virus neutralizing antibody titers in mouse serum at 7 days after vaccination with the three vaccines (A); viral RNA levels in brain tissue (B) and spinal cord (C) at different time points after lethal rabies virus challenge in mice, comparison of body weight changes (D) and survival rates (E) of mice in different groups. ns or unmarked: not significant; *: p<0.05; **: p<0.01; ***: p<0.001; ****: p<0.0001.

### SPECIFIC IMPLEMENTATIONS

To further elaborate on the technical means adopted in the present invention and their effects, the present invention is further described below with reference to specific examples of rabies vaccine construction. It should be understood that the specific embodiments described herein are for illustrative purposes only and are not intended to limit the scope of the present invention.

In the description herein, for the sake of brevity, a protein and its encoding gene (DNA) are sometimes used interchangeably. Those skilled in the art should understand that they represent different substances in different contexts and can easily comprehend their meanings based on the context.

Similarly, for the sake of brevity, RNA such as mRNA and its encoding gene (DNA) are sometimes used interchangeably. Those skilled in the art should understand that they represent different substances in different contexts and can easily comprehend their meanings based on the context.

In the examples of the present invention, if no specific temperature is specified for experimental operations, the temperature generally refers to room temperature (10-30 °C).

The addition amounts, contents and concentrations of various substances mentioned herein refer to mass percentages unless otherwise specified.

For the techniques or conditions not specified in the examples, conventional methods are adopted, which are performed according to the techniques or conditions described in the literature in the art or in accordance with product instructions. The reagents or instruments used are conventional products commercially available through formal channels.

### Materials and Methods

The immunogen design and screening, mRNA preparation, mRNA vaccine preparation, animal immunization protocols, and detection methods involved in the experiments in the examples are as follows:

### I. Immunogen Design

The rabies virus genome encodes five major proteins: N, P, M, G, and L. The L protein (Large Protein) is the RNA-dependent RNA polymerase of rabies virus, which is critical for rabies viral replication and transcription. Although RNA viruses have a relatively high mutation rate, mutations in proteins essential for the viral life cycle (such as the L protein) are usually restricted, as any mutation adversely affecting its function may reduce viral viability. In the present invention, L protein sequences from five vaccine strains (CVS-11, aG, CTN, ERA, PM1503) are selected as the source for LT antigen design, and conserved dominant T-cell epitope-enriched regions are screened and obtained using these sequences as targets.
i. Search and download the L protein sequences of the five vaccine strains from the Genebank database, including PM (GenBank: DQ099525.1), CTN-1 (GenBank: FJ959397.1), aG (GenBank: GQ412744.1), ERA (GenBank: EF206707.1), and CVS-11 (GenBank: GQ918139).
ii. Use the prediction tools NetMHCpan and NetMHCIIpan, and select 12 MHC-I and 54 MHC-II supertypes covering more than 90% of the human population as the prebinding HLA types.
iii. Analyze the five L protein antigen sequences one by one, screen out MHC-I and MHC-II epitopes with high binding affinity respectively, and map the potential dominant epitopes to the original sequences.
iv. Integrate the analysis of dominant epitope-enriched regions and highly similar regions among the five proteins, and truncate and preliminarily screen the T-cell antigen sequence of the LT protein.
v. Exclude the allergenic and toxic characteristics of the T-cell antigen using the tools ALLERGENFP V1.0, ALLERGENFP V2.0, and allercatpro, and finally obtain the recombinant L protein LT (1035 amino acid residues in length).

The obtained recombinant LT protein sequence is inserted between the BamHI and AgeI multiple cloning sites of the high-expression vector in the optimal combination described in Chinese Patent Application No. 202211129466.2, and a Flag tag is added to its 3' end. Sequence optimization is performed according to eukaryotic codon usage bias and RNA secondary structure. The corresponding DNA template sequence of the coding region is shown in SEQ ID NO: 6, which was synthesized by Shanghai JieRui BioEngineering Co., Ltd. and designated RABV-LT. This sequence is inserted into the high-expression vector, and the corresponding DNA template sequence for mRNA preparation is shown in SEQ ID NO: 10.

In addition, the antigen sequence for inducing neutralizing antibody production is constructed using the DNA sequence of the glycoprotein (RABV-G) of the Pitman-Moore (PM) vaccine strain, and optimized according to eukaryotic codon usage bias. The corresponding DNA template sequence of the coding region is shown in SEQ ID NO: 3; the corresponding DNA template sequence for mRNA preparation is shown in SEQ ID NO: 11.

### II. Construction of Different Dual-Antigen RABV-G-LT Molecules

Using the high-expression vector in the optimal combination described in Chinese Patent Application No. 202211129466.2 as a template, the elements from the N-terminus to the C-terminus downstream of the T7 promoter include: 5'-UTR sequence, coding sequence, 3'-UTR sequence, poly(A) tail sequence, and linearization restriction site XbaI. The coding sequence includes, from 5' end to 3' end, the first protein domain RABV-G sequence as set forth in SEQ ID NO: 3; the IRES element sequence as set forth in SEQ ID NO. 7; the second protein domain RABV-LT sequence as set forth in SEQ ID NO: 6; and a degradation domain sequence, either estrogen receptor (ER) domain mutant 2 as set forth in SEQ ID NO: 8, or dihydrofolate reductase (DHFR) as set forth in SEQ ID NO: 9. The different dual-antigen RABV-G-LT molecules in the above order are named as follows: RABV-G-IRES-LT, with the corresponding DNA template sequence for mRNA preparation as set forth in SEQ ID NO: 12; RABV-G-IRES-LT-ERmut2, with the corresponding DNA template sequence for mRNA preparation as set forth in SEQ ID NO: 13; RABV-G-IRES-LT-DHFR, with the corresponding DNA template sequence for mRNA preparation as set forth in SEQ ID NO: 14.

### III. Preparation of mRNA Vaccines

The different dual-antigen RABV-G-LT expression vectors constructed in Section II above were linearized using restriction endonuclease XbaI. The corresponding modified mRNAs were synthesized using an in vitro transcription kit and a capping kit purchased from Novoprotein Scientific Inc., with 1-methyluridine replacing uridine, following the manufacturer's instructions. The mRNAs were purified by lithium chloride precipitation, washed three times with 75% ethanol, air-dried, dissolved in RNase-free water, and quantified using a NanoDrop spectrophotometer. The prepared mRNA molecules were submitted to a company to formulate mRNA-lipid nanoparticle (LNP) vaccines for subsequent mouse experiments.

### IV. Animal Immunization Protocols

First immunization experiment: To evaluate the immunogenicity of recombinant polymerase large protein L RABV-LT, female BALB/c mice aged 6-8 weeks were divided into 2 groups (n=15 per group; 10 for protective efficacy assessment after challenge, 5 for cellular immune response detection): an empty LNP negative control group and a group immunized with 3 µg of RABV-LT mRNA. Vaccination was administered intramuscularly into the thigh muscle of one hind leg at a volume of 50 µL. Seven days later, the mice were challenged intramuscularly in the contralateral hind leg with 40 MLD₅₀ of rabies virus CVS-11.

Second immunization experiment: To evaluate the immunogenicity of different dual-antigen RABV-G-LT mRNA vaccines in BALB/c mice, female BALB/c mice aged 6-8 weeks were divided into 4 groups (n=6 per group): an empty LNP negative control group, a RABV-G-IRES-LT mRNA vaccine group, a RABV-G-IRES-LT-ERmut2 mRNA vaccine group, and a RABV-G-IRES-LT-DHFR mRNA vaccine group. All vaccines were administered intramuscularly at a dose of 3 µg in a single volume of 100 µL (see Table 1). Blood samples were collected 12 days after immunization to analyze serum RABV-G-specific antibody levels. In addition, 3 mice from each group were randomly selected for ELISpot assays to analyze T-cell responses.

**Table 1**

| Group | Dose | Immunization Route |
|---|---|---|
| Empty LNP negative control group | Nucleic acid-free cationic lipid nanoparticles, equal volume to the test groups | Intramuscular injection |
| RABV-G-IRES-LT group | 3 µg | Intramuscular injection |
| RABV-G-IRES-LT-ERmut2 group | 3 µg | Intramuscular injection |
| RABV-G-IRES-LT-DHFR group | 3 µg | Intramuscular injection |

The third immunization experiment was designed to evaluate the protective efficacy of the dual-antigen RABV-G-LT mRNA vaccine, the RABV-G mRNA vaccine, and a commercial inactivated vaccine against lethal rabies virus challenge at 7 days post-vaccination.

Female BALB/c mice were divided into 4 groups with 14 mice per group: an empty LNP negative control group (50 µL), a single-dose 3 µg RABV-G mRNA vaccine group, a single-dose 3 µg dual-antigen RABV-G-LT mRNA group, and a single-dose commercial inactivated vaccine group (potency: 1 IU/mL). All vaccines were administered intramuscularly into the thigh muscle of one hind leg at a volume of 50 µL. Seven days later, the mice were challenged intramuscularly in the contralateral hind leg with 40 MLD₅₀ of rabies virus strain CVS-11. On day 7 (7 dpi) and day 12 (12 dpi) after challenge, 3 mice were randomly selected from each group to collect brain and spinal cord tissues for the detection of viral RNA levels, as shown in Table 2 below.

**Table 2**

| Group | Dose | Immunization Route | Brain/Spinal Cord Collection at Different Time Points Post-Challenge |
|---|---|---|---|
| Empty LNP negative control group | 50 µL of nucleic acid-free cationic lipid nanoparticles | Intramuscular injection | 3 mice/group |
| | | | 7 dpi. |
| RABV-G mRNA (3 µg) | 3 µg | Intramuscular injection | 3 mice/group |
| | | | 7 dpi., 12 dpi. |
| RABV-G-LT mRNA (3 µg) | 3 µg | Intramuscular injection | 3 mice/group |
| | | | 7 dpi., 12 dpi. |
| Inactivated vaccine (1 IU/mL) | 3 µg | Intramuscular injection | 3 mice/group |
| | | | 7 dpi.,12 dpi. |

### V. Detection Methods

### Blood Collection

Mice were bled before primary immunization on day 1, followed by orbital venous plexus bleeding at scheduled time points, and mice were euthanized by cervical dislocation at the end of the experiment.

Collected mouse whole blood was placed into 1.5 mL sterile EP tubes, allowed to stand at 37 °C for 1 h, and then kept at 4 °C for 20 min to allow natural clotting. Clotted mouse serum was centrifuged at 7500 g for 20 min. The serum was then inactivated in a 56 °C water bath for 30 min to eliminate complement activity; a small portion was reserved for immediate use, and the remainder was stored at -80 °C until use.

### Detection of Target Protein Expression by Western Blot

i. Separation gels of appropriate concentrations were prepared according to the molecular weight of the target protein, and SDS-polyacrylamide gel electrophoresis (SDS-PAGE) was performed.
ii. A total of 20 µL of prepared samples was loaded in the order of the samples to be tested. Electrophoresis was run at 80 V for 30 min, then the voltage was adjusted to 100 V, and electrophoresis was continued for 1.5 h. The termination of electrophoresis was determined based on the position of the bromophenol blue band.
iii. Membrane transfer: Wet transfer was used. First, the PVDF membrane was activated in methanol for 30 s. Sponges, filter papers, and the PVDF membrane were soaked in transfer buffer, and assembled in the order: cathode plate (black) → sponge → filter paper → gel → PVDF membrane → filter paper → sponge → anode plate (white). Transfer was performed at a constant current of 200 mA for 1.5-2 h.
iv. After transfer, the PVDF membrane was blocked with 5% skimmed milk for 1-2 h.
v. Primary antibody incubation: Specific antibody was added at a dilution of 1:1000 (w/v), and the membrane was incubated on a shaker at room temperature for 2 h, then washed 5 times with PBST with shaking for 3 min each time.
vi. Secondary antibody incubation: HRP-conjugated IgG antibody was added at a dilution of 1:5000 (w/v), and the membrane was incubated at room temperature for 1 h, then washed 5 times with PBST with shaking for 3 min each time.
vii. Color development: Chromogenic reagent was added and incubated for 1 min. The membrane was exposed for 2 min using an imaging analyzer, and the results were recorded and analyzed.

### Detection of RABV-G Binding Antibody Titer by Enzyme-Linked Immunosorbent Assay (ELISA)

i. RABV-G protein was diluted to 1 ng/µL with coating buffer (50 mM carbonate buffer, pH 9.6), added at 100 µL per well, and incubated overnight at 4 °C.
ii. The plate was washed once with PBST (containing 0.05% Tween-20), patted dry, and blocked with 200 µL per well of 5% skimmed milk at room temperature for 2 h.
iii. Serum samples were diluted 100-fold in the first well using 5% skimmed milk as the diluent, followed by 2-fold serial dilutions, added to the reaction wells, and incubated at 37 °C for 1 h.
iv. The plate was washed 5 times with PBST, patted dry, and incubated with 100 µL per well of HRP-conjugated goat anti-mouse IgG antibody (1:5000) at room temperature for 1 h.
v. After the reaction, the plate was washed 6 times with PBST, patted dry, and OPD chromogenic solution (one gold tablet and one silver tablet dissolved in 20 mL deionized water) was added in the dark, and the reaction was carried out for 5-10 min.
vi. The reaction was stopped with 2 M sulfuric acid stop solution. The absorbance was read at 490 nm using a microplate reader.
vii. Data were analyzed. The cut-off value was set at 2.1-fold the reading of the negative control group. The endpoint dilution titer of RABV-G-specific binding antibody was calculated and expressed as the geometric mean titer (GMT). GraphPad Prism 9 software was used for plotting.

### Detection of Serum Live Virus Neutralizing Antibody Titer by Fluorescent Antibody Virus Neutralization Test (FAVN)

The assay was performed according to the standard operating procedure of the OIE Reference Laboratory for Rabies in the United Kingdom. First, 100 µL of complete DMEM medium was added to each well. Then 50 µL of pre-inactivated serum sample was added to the first column, mixed well using a multi-channel pipette, and 50 µL was transferred to the second column. Serial dilutions were performed to the last column in the same manner, and 50 µL was discarded after mixing. Another 96-well plate was used as the control plate, on which standard positive serum (0.5 IU/mL) and standard negative serum (non-immune mouse serum) were diluted in the same manner. Virus back-titration wells, virus control wells, cell control wells, and medium control wells were also set. Then 50 µL of 100 TCID₅₀ rabies virus (CVS-11 strain) was added to each well, while cell and medium control wells were added with only complete DMEM medium without virus. Plates were incubated at 37 °C with 5% CO₂ for 1 h. Subsequently, 50 µL of BHK-21 cell suspension at a density of 4×10⁵ cells/mL was added to each well, and cells were not added to medium control wells. Plates were incubated at 37 °C with 5% CO₂ for 48 h. After incubation, the cell supernatant was discarded, and fixation and staining were performed. Cells were fixed with 80% acetone for 30 min, the fixative was removed, and plates were air-dried. FITC-conjugated anti-rabies nucleocapsid protein antibody and 50 µL of 1% Evans blue staining solution were added to each well, followed by incubation at 37 °C in the dark for 45-60 min. Fluorescence microscopy was used to determine the presence of one or more fluorescent cells, and wells were qualitatively scored as "positive" (infected, with fluorescence) or "negative" (no fluorescence). Serum titer was defined as the dilution that neutralized 100% of the virus in 50% of wells (logD₅₀). A titer of 0.5 IU/mL was the neutralization standard recommended by WHO. Under the same experimental conditions, the 50% neutralizing dilution ratios of the test serum and the OIE reference serum against the quantified virus were calculated separately. The rabies neutralizing titer of the test serum was obtained by comparing the two ratios and multiplying by the titer of the standard serum (0.5 IU/mL). Titers were expressed as the geometric mean titer (GMT) of neutralizing titers, and GraphPad Prism 9 software was used for plotting.

Analysis of RABV-G/RABV-LT Antigen-Specific T Cells by Intracellular Cytokine Staining (ICS)
i. Mouse splenocytes were isolated at different time points after immunization and prepared into a single-cell suspension. Spleens were harvested after euthanasia, ground into single cells in R10 medium, and centrifuged at 500 g for 5 min. The supernatant was discarded, and the cell pellet was resuspended. Then 5 mL of mouse red blood cell lysis buffer was added, mixed gently, and incubated at room temperature for 5-10 min. Lysis was terminated by adding 10 mL of R10 medium, followed by centrifugation at 800 g for 3 min. The supernatant was discarded, cells were washed once with 5 mL of R10 medium, resuspended in 5 mL of R10 medium, and counted.
ii. Peptide stimulation: 2×10⁶ cells per well were plated in 96-well plates and stimulated with rabies virus glycoprotein peptide pool at a final concentration of 1 µg/mL. The negative control group was treated with DMSO, and the positive control group was treated with 50 ng/mL PMA and 5 µg/mL Ionomycin. Plates were incubated at 37 °C with 5% CO₂ for 1 h.
iii. BD GolgiStop^{™} Protein Transport Inhibitor (diluted 1:1000) was added, and plates were incubated at 37 °C incubator for 5 h to complete stimulation. Cells were centrifuged at 500 g for 5 min, washed once with PBS, and subjected to fluorescent antibody staining (staining volume: 50 µL for all steps, same below).
iv. Surface staining was performed using Zombie Aqua^{™}, Mouse CD4-AF700, Mouse CD8a-FITC, and Mouse CD3 PerCP-Cy5.5 at room temperature in the dark for 20 min. Cells were washed once with PBS and centrifuged at 500 g for 5 min.
v. Cells were fixed with Cytofix/Cytoperm at 4 °C in the dark for 20 min, washed once with PBS, and centrifuged at 1000 g for 5 min.
vi. Permeabilization: 10× BD Perm/Wash^{™} buffer was diluted to 1× working solution with ultrapure water. Cells were resuspended in 1× BD Perm/Wash^{™} buffer at 200 µL per well, incubated at room temperature in the dark for 15 min, and centrifuged at 1000 g for 5 min. The supernatant was carefully discarded.
vii. Cells were stained with Mouse IFN-γ-PE, Mouse IL-2-APC, and Mouse TNF-α-BV605 diluted in 1× BD Perm/Wash^{™} buffer at 4 °C for 30 min. Data were acquired using a BD LSRFortessa flow cytometer; at least 30,000 CD8⁺ T cells were collected per sample, followed by data analysis using FlowJo software.

### Mouse IFN-γ ELISpot Assay

i. Antibody coating was performed using the BD ELIspot Mouse IFN-γ ELISpot Kit. Anti-IFN-γ antibody was diluted 1:200 (w/v) in sterile PBS according to the required number of wells, and added to ELISpot plates at 100 µL per well. (Antibody dilution volume: number of experimental wells 110 µL). Plates were incubated overnight at 4 °C.
ii. The overnight-incubated ELISpot plates were taken out, the liquid in the wells was discarded, and 200 µL of R10 medium was added to wash once. After standing for 3 min, the solution was removed. Then 200 µL of fresh R10 medium was added, followed by blocking at room temperature for 2 h.
iii. After blocking, the medium was discarded. Stimulant (stimulatory peptide pool, final concentration of single peptide 5 µg/mL) was added at 50 µL per well; R10 medium served as the negative control, and PMA (50 ng/mL) + Ionomycin (1 µg/mL) or other positive stimulants served as the positive control. Cell concentration was adjusted to 4×10⁶ cells/mL, and 50 µL of cell suspension was added to each well in sequence. After gentle mixing, plates were placed in a humid chamber and incubated in a CO₂ incubator for approximately 20 h. The first three steps were performed in a biological safety cabinet; subsequent steps did not require aseptic conditions. The remaining cells were cryopreserved: centrifuged at 500 g for 5 min, the supernatant was discarded, and 2 mL of commercial freezing medium was added; each sample was divided into 2 aliquots.
iv. After incubation, the culture was discarded. Each well was washed twice with 200 µL of pre-chilled distilled water and three times with 200 µL of PBST. The liquid was discarded after standing for 3-5 min each time, and residual liquid was patted dry on absorbent paper (the plate should be kept moist during washing and not allowed to dry out).
v. Biotin-conjugated detection antibody was diluted 1:250 (w/v) in antibody diluent (PBS + 10% FBS), added at 100 µL per well, and incubated at room temperature for 2 h. The liquid was discarded, and each well was washed four times with 200 µL of PBST. The liquid was discarded after standing for 1-2 min each time, and residual liquid was patted dry on absorbent paper.
vi. Streptavidin-HRP was diluted 1:100 (w/v) in antibody diluent (PBS + 10% FBS) and added at 100 µL per well, followed by incubation at room temperature for 1 h. The reaction solution was discarded, and each well was washed 5 times with 200 µL of PBST; the liquid was discarded after standing for 1-2 min each time. Finally, each well was washed 3 times with 200 µL of PBS, and the liquid was discarded.
vii. Color development: AEC chromogenic solution was prepared immediately before use. One drop (approximately 20 µL) of chromogenic substrate was added to 1 mL of chromogenic buffer, mixed thoroughly, and added at 100 µL per well. The reaction was carried out at room temperature in the dark for 5-60 min. Color development was monitored; when clear red spots appeared, the plate was gently rinsed with tap water for 5 min to stop the reaction. (Short color development time results in pale positive spots; excessive time leads to high background, which can be judged with reference to positive and negative control wells). Plates were air-dried or blower-dried.
viii. Data acquisition. The 96-well plate was placed in an ELISpot reader to count positive spots.

### Description of the T-Cell Epitope Peptide Library for RABV-LT Recombinant Protein

Sequence alignment between the RABV-LT recombinant protein and the full-length L protein revealed that amino acids 600-950 constitute the RNA-dependent RNA polymerase functional domain, which performs capping enzyme activity. Therefore, in the present invention, this region was covered using an overlap method (11-amino acid overlap), yielding a total of 85 T-cell epitope peptides synthesized by Qiangyao Biotechnology Co., Ltd.

Peptide pools: Based on the aforementioned single peptides arranged in amino acid order, 10 peptide pools were established. Pools 1 to 5 each contained 9 single peptides, and pools 6 to 10 each contained 8 single peptides.

### RNA Extraction and Detection in Mouse Brain and Spinal Cord

i. Mice at different time points post-infection were euthanized by cervical dislocation, and brain and spinal cord tissues were collected. Each brain was divided into two parts: one part was fixed in 4% paraformaldehyde, transferred to 75% ethanol after 24 h, and sent to a company for paraffin sectioning and HE staining; the other brain part and spinal cord tissue were each added to 1 mL of pre-chilled RNAzol^{®} RT reagent (Molecular Research Center) and fully homogenized using a tissue sonicator (65 Hz, 30 s, twice).
ii. Homogenized samples were centrifuged at 18000 g for 10 min at 4 °C. The supernatant was transferred to a new EP tube; 200 µL of each supernatant was used for total RNA extraction, and the remainder was stored at -80 °C until use.
iii. Anhydrous ethanol was added at a 1:1 volume ratio to the tissue supernatant, mixed thoroughly by vortexing, and centrifuged at 18000 g for 2 min at 4 °C. The supernatant was transferred to an adsorption column.
iv. Centrifugation was performed at 18000 g for 2 min at 4 °C, followed by resuspension. Then 400 µL of Prewash buffer was added, centrifuged at 18000 g for 1 min at 4 °C, and washed twice.
v. Then 700 µL of RNAwash buffer was added, centrifuged at 18000 g for 2 min at 4 °C, followed by a dry spin.
vi. The RNA was eluted into an RNase-free EP tube with 50 µL of RNase-free water, centrifuged at 18000 g for 2 min at 4 °C, and the total RNA concentration was determined.

### Quantitative Real-Time PCR (qPCR)

A One Step RT-PCR Kit (Vazyme) was used in this experiment to perform reverse transcription and PCR in a single system, minimizing experimental operation errors. The reaction mixture was prepared first. For amplification of the RABV nucleocapsid protein gene, the forward primer N-F: AATGCGACGGTTATTGCTGC; reverse primer N-R: TGCCACGTCGGTCTTTGTTA. Total RNA concentration of each sample was determined, and 50 ng was used per reaction. The reaction system is shown in Table 3.

**Table 3**

| Reagent | Volume (µL) |
|---|---|
| Nuclease-free water | Make up to 20 µL |
| 2× One Step Mix | 10 |
| One Step Enzyme Mix | 1 |
| Forward Primer (10 µM) | 0.4 |
| Reverse Primer (10 µM) | 0.4 |
| Template RNA | 50 ng |
| Total | 20 |

After preparing the reaction mixture in an EP tube (4 replicate wells per sample), the mixture was added to a 96-well PCR plate, and the reaction program (40 cycles) was set as shown in Table 4:

**Table 4**

| Stage | Reps | Temp (°C) | Time |
|---|---|---|---|
| Reverse transcription | 1 | 55 | 15 min |
| Pre-denaturation | 1 | 95 | 30 |
| PCR template denaturation | 40 | 98 | 15 s |
| Annealing | 40 | 60 | 20 s |

After the reaction was completed, the amplification curves of real-time PCR were collected and exported for data analysis.

The present patent is further described in detail below with reference to the drawings and specific experiments. Unless otherwise specified, the reagents, instruments, equipment, and methods used in this patent are all commercially available conventional reagents, instruments, equipment, and methods in the art.

### Example 1: Construction of recombinant T-cell antigen RABV-LT and verification of its expression via mRNA transfection into HEK293T cells

The recombinant T-cell antigen RABV-LT of the L protein was constructed according to Experimental Method I as described above, with a Flag tag added to its 3' end, as shown in panel A of FIG. 1.

RABV-LT mRNA was prepared according to Experimental Method III as described above and transfected into HEK293T cells for expression verification. The mRNA and the transfection reagent Lipofectamine 3000 were mixed at a mass ratio of 1:3, and samples were collected 24 h after transfection. Western blot analysis was performed using an anti-Flag tag antibody as the primary antibody.

The results are shown in panel B of FIG. 1. High-level expression of RABV-LT was detected in HEK293T cells after transfection with RABV-LT mRNA. The target band was observed at approximately 120 kDa, consistent with the expected molecular weight.

### Example 2: Evaluation of immunogenicity of RABV-LT mRNA vaccine in BALB/c mice

In this example, RABV-LT mRNA with verified correct expression in Example 1 was used to prepare a cationic lipid nanoparticle vaccine encapsulating RABV-LT mRNA for animal experiments.

BALB/c mice were immunized with the prepared mRNA-RABV-LT vaccine at a dose of 3 µg mRNA-loaded cationic lipid nanoparticle vaccine per mouse. The negative control group received an equal volume of nucleic acid-free cationic lipid nanoparticles. Mice were sacrificed one week after immunization, and splenocytes were isolated. The antigen-specific T-cell responses were detected by analyzing the secretion of RABV-LT-antigen-specific T-cell cytokines via intracellular cytokine staining (ICS) according to Experimental Method V as described above.

As shown in panels A and B of FIG. 2, compared with the control group, mice immunized with the RABV-LT mRNA vaccine exhibited significantly higher levels of IFN-γ and TNF-α secretion in both RABV-LT-specific CD4⁺ T cells and CD8⁺ T cells. Specifically, the average proportion of IFN-γ CD4⁺ T cells was 0.6% (vs. 0.2% in the negative control group; panel B of FIG. 2); the average proportion of IFN-γ CD8⁺ T cells was 1.25% (vs. 0.45% in the negative control group); and the proportion of TNF-α CD8⁺ T cells (0.61%) was also significantly higher than that in the negative control group (0.28%) (p<0.05; panel A of FIG. 2).

These results indicate that immunization with the RABV-LT vaccine effectively induces antigen-specific T-cell responses with secretion of multiple cytokines, suggesting that the vaccine has strong viral clearance activity and great potential in protecting against rabies virus infection.

### Example 3: Protective efficacy of RABV-LT mRNA vaccine against rabies virus challenge in mice

To further evaluate the in vivo protective effect of the RABV-G mRNA nucleic acid vaccine of the present invention, BALB/c mice were used as a model with 10 mice per group in this example. Mice were intramuscularly inoculated with 3 µg of RABV-LT mRNA or empty LNP, with a total inoculation volume of 50 µL per mouse. Seven days after immunization, mice were challenged intramuscularly (in the contralateral leg) with 5× 10^{3.5} TCID₅₀ of the fixed rabies virus strain CVS-11. Body weight changes and survival status were monitored continuously for 15 days post-infection.

As shown in panels A and B of FIG. 3, the RABV-LT mRNA vaccine conferred a 70% protection rate against the lethal viral challenge (panel A of FIG. 3). Mice in the unvaccinated empty vector control group showed a sharp decline in body weight starting from day 4 post-infection and all died by day 9. In contrast, mice in the RABV-LT mRNA vaccine group showed a slight weight loss immediately after infection, followed by a gradual recovery. Body weight declined slowly to a trough from day 4 to day 9, with 2 mice dying; weight then recovered gradually, with one additional mouse dying on day 13. All remaining mice survived normally until the end of the 15-day monitoring period, with body weight fully restored.

These results demonstrate that a single inoculation of RABV-LT mRNA vaccine provides moderate but effective protection against rabies virus infection in mice.

### Example 4: Construction and Verification of a Dual-Antigen RABV-G-LT mRNA Vaccine Incorporating RABV-LT Antigen and RABV-G Antigen

Given that the monovalent RABV-LT mRNA vaccine provided a certain level of protection in the above examples, and monovalent RABV-G mRNA also exhibited excellent protective efficacy, in this example RABV-G and RABV-LT were incorporated into a single antigen molecule. This design allows the induction of B-cell immunogen expression on the one hand, and enables normal expression of T-cell antigens on the other, thereby achieving rapid induction of both neutralizing antibody responses and cellular immune responses. For this purpose, RABV-LT and RABV-G were linked via an internal ribosome entry site (IRES) sequence to construct a dual-antigen RABV-G-LT mRNA rabies vaccine capable of independent and efficient expression of both antigens. In addition, to improve the presentation of T-cell antigens, degradation domains including mutant estrogen receptor 2 (ERmut2) and dihydrofolate reductase (DHFR) were fused to the C-terminus of the RABV-LT sequence, enabling self-degradation of RABV-LT into multiple T-cell epitopes. The constructed dual-antigen RABV-G-LT molecules were RABV-G-IRES-LT, RABV-G-IRES-LT-ERmut2 fused with ERmut2, and RABV-G-IRES-LT-DHFR fused with DHFR.

The three different dual-antigen RABV-G-LT mRNA constructs were prepared according to Experimental Method III as described above, and transfected separately into HEK293T cells for expression verification. At 16 h post-transfection, the proteasome inhibitor MG132 was added to a final concentration of 50 µM to wells transfected with RABV-G-IRES-LT and RABV-G-IRES-LT-ERmut2. Trimethoprim (TMP), which inhibits the degradation function of DHFR, was used at a working concentration of 50 µM and added to cells transfected with RABV-G-IRES-LT-DHFR. Samples were harvested 8 h later, and expression was verified by Western blot using anti-RABV-G and anti-flag antibodies as primary antibodies. As shown in panels A and B of FIG. 4, in the RABV-G-IRES-LT and RABV-G-IRES-LT-ERmut2 groups, the protein band of the LT recombinant antigen was significantly weaker in MG132(-) wells than in MG132(+) wells, whereas distinct RABV-G protein bands were observed in both groups. Moreover, the addition of MG132(+) also enhanced RABV-G expression. Furthermore, in the RABV-G-IRES-LT-DHFR group, the addition of TMP greatly increased the intracellular expression of RABV-G protein and induced detectable expression of the LT recombinant antigen from an undetectable level.

These results demonstrate that IRES-mediated ligation enables efficient and independent expression of RABV-G and RABV-LT, and supports the incorporation of RABV-LT antigen and RABV-G antigen into a single dual-antigen RABV-G-LT molecule.

### Example 5: Comparison of Immunogenicity of Different Dual-Antigen RABV-G-LT mRNA Vaccines in BALB/c Mice

This example further investigated the differences in the immunological effects of different dual-antigen RABV-G-LT mRNA vaccines in vivo. The three mRNA constructs from Example 4 were formulated into liposomal nanoparticle vaccines. BALB/c mice were immunized according to the Group 2 immunization protocol described in Experimental Method IV, with 6 mice per group, receiving 3 µg via intramuscular inoculation, and empty vector as the control group. Blood was collected from the orbital venous plexus 12 days after primary immunization, and RABV-G-binding antibody levels were evaluated by ELISA. Meanwhile, splenocytes were isolated from 3 randomly selected mice per group, stimulated with G-protein peptide pool and LT peptide pool respectively, and T-cell immune responses were measured by ELISpot.

As shown in panel A of FIG. 5, compared with the empty vector control group, the RABV-G-IRES-LT, RABV-G-IRES-LT-DHFR, and RABV-G-IRES-LT-ERmut2 groups all induced high levels of RABV-G-binding antibodies, with no significant differences among the three groups. The ELISpot results in panel B of FIG. 5 show that the mean numbers of IFN-γ spot-forming cells (SFC) per 10⁶ lymphocytes in response to the G-protein peptide pool in splenocytes of the mice were 347, 410, and 525 for the RABV-G-IRES-LT, RABV-G-IRES-LT-DHFR, and RABV-G-IRES-LT-ERmut2 groups, respectively; experimental groups fused with degradation domains exhibited stronger immune responses stimulated by the G-protein peptide pool. For responses stimulated by the LT recombinant antigen peptide pool, the mean number of IFN-γ SFC per 10⁶ lymphocytes was the lowest in the RABV-G-IRES-LT group (only 65), the highest in the RABV-G-IRES-LT-DHFR group (193), and 98 in the RABV-G-IRES-LT-ERmut2 group.

These results demonstrate that the RABV-G-IRES-LT-DHFR design not only stimulates potent RABV-G-specific IgG binding antibody titers but also allows independent translation of the RABV-LT antigen, which is cleaved into multiple epitopes via the DHFR degradation peptide, thereby effectively inducing cellular immune responses. Therefore, RABV-G-IRES-LT-DHFR was selected as the immunogen for subsequent dual-antigen mRNA vaccine studies, and is abbreviated as RABV-G-LT.

### Example 6: Comparison of Protective Efficacy of Dual-Antigen RABV-G-LT mRNA Vaccine, RABV-G mRNA Vaccine, and Commercial Inactivated Vaccine Against Lethal Rabies Virus Challenge 7 Days Post-Inoculation

Given that the monovalent RABV-LT mRNA vaccine provided 70% protection against challenge 7 days after inoculation in Example 3, this example further explored the protective efficacy of the dual-antigen RABV-G-LT mRNA vaccine against challenge 7 days post-inoculation. According to the Group 3 immunization protocol described in Experimental Method IV, BALB/c mice were used as a model, with a commercial inactivated vaccine at an effective dose of 1 IU/mL as the experimental control, to evaluate the protective levels of monovalent RABV-G mRNA vaccine and RABV-G-LT mRNA vaccine. A total of 5 groups of mice (14 mice per group) were included, and mRNA vaccine groups received 3 µg via intramuscular inoculation on day 0. On day 7, 5 mice were randomly selected from each group for blood collection to detect live virus neutralizing antibody titers. As shown in panel A of FIG. 6, the geometric mean titer of live virus neutralizing antibody was 4.3 IU/mL in the RABV-G mRNA vaccine group, 1.3 IU/mL in the inactivated vaccine group, and the lowest (0.42 IU/mL) in the RABV-G-LT mRNA vaccine group, with one mouse failing to meet the WHO standard (0.5 IU/mL).

Subsequently, the in vivo protective efficacy of the three vaccines against lethal rabies virus challenge at an early time point after inoculation was further compared. On day 7 post-inoculation, mice were challenged intramuscularly with 5×10^{3.5} TCID₅₀ of lethal CVS-11 rabies virus. On day 7 and day 12 post-challenge, 3 mice were randomly selected from each group to extract RNA from brain tissue and spinal cord for relative quantitative PCR detection. Body weight changes and survival status of each mouse were monitored continuously for 20 days post-infection.

As shown in panels B and C of FIG. 6, high levels of rabies virus RNA were detected in the brain and spinal cord of all mice in the empty vector control group on day 7 post-infection; no samples could be collected on day 12 as all control mice had died. In contrast, all vaccinated groups exhibited low viral RNA levels. Compared with the Blank group (uninfected, unvaccinated), the viral RNA levels in brain tissue on day 7 post-challenge were 5.28-fold, 17-fold, and 57.6-fold higher in the RABV-G-LT mRNA vaccine, RABV-G mRNA vaccine, and commercial inactivated vaccine groups, respectively. Viral RNA levels decreased on day 12 post-infection, with very low but detectable levels in the RABV-G-LT group. Similarly, for viral RNA levels in the spinal cord on day 7 and day 12, the commercial inactivated vaccine group showed the highest levels, followed by the RABV-G mRNA vaccine group, and the RABV-G-LT mRNA vaccine group showed the lowest levels.

Body weight changes and survival status of mice in each group post-infection were monitored. As shown in panels D and E of FIG. 6, mice in the empty vector control group began losing weight on day 1, partially recovered, then declined sharply from day 4, and all died by day 9. Mice in the three vaccinated groups showed slight weight loss immediately after infection followed by gradual recovery. From day 4 onward, the inactivated vaccine control group exhibited slow weight loss reaching a trough on day 15, then recovered gradually; mice in the mRNA vaccine groups showed steady slow weight gain with minor fluctuations. At the end of the 20-day monitoring period, all mice in the RABV-G-LT mRNA vaccine and RABV-G mRNA vaccine groups survived normally with restored body weight, whereas the inactivated vaccine group showed only a 62.5% survival rate.

These results indicate that the RABV-G-LT mRNA vaccine can more effectively clear the virus and control rabies virus infection.

Taken together, the above examples show that the dual-antigen RABV-G-LT mRNA vaccine provided in the present invention can rapidly induce immune responses post-inoculation. Before substantial maturation and accumulation of protective antibodies, it activates effective cellular immune responses to better suppress viral replication, exhibiting broad application prospects in the prevention and treatment of rabies virus infection.

### Sequence Listing

1. SEQ ID NO: 1: Full-length amino acid sequence of rabies virus structural protein glycoprotein (G)
   (omitted)
2. SEQ ID NO: 2: Amino acid sequence of the extracellular domain of rabies virus structural protein glycoprotein (G)
   (omitted)
3. SEQ ID NO: 3: Codon-optimized full-length nucleic acid sequence of rabies virus structural protein glycoprotein (G)
   (omitted)
4. SEQ ID NO: 4: Codon-optimized nucleic acid sequence of the extracellular domain of rabies virus structural protein glycoprotein (G)
   (omitted)
5. SEQ ID NO: 5: Amino acid sequence of RABV-LT protein
   (omitted)
6. SEQ ID NO: 6: DNA nucleic acid sequence of RABV-LT after codon and RNA secondary structure optimization
   (omitted)
7. SEQ ID NO: 7: Nucleic acid sequence of the internal ribosome entry site derived from encephalomyocarditis virus
   (omitted)
8. SEQ ID NO: 8: Sequence of estrogen receptor (ER) domain mutant 2
   (omitted)
9. SEQ ID NO: 9: Sequence of dihydrofolate reductase (DHFR)
   (omitted)
10. SEQ ID NO: 10: DNA template sequence for preparing RABV-LT mRNA
   (omitted)
11. SEQ ID NO: 11: DNA template sequence for preparing RABV-G mRNA
   (omitted)
12. SEQ ID NO: 12: DNA template sequence for preparing RABV-G-IRES-LT mRNA
   (omitted)
13. SEQ ID NO: 13: DNA template sequence for preparing RABV-G-IRES-LT-ERmut2 mRNA
   (omitted)
14. SEQ ID NO: 14: DNA template sequence for preparing RABV-G-IRES-LT-DHFR mRNA
   (omitted)

## Claims

1. An immunogenic composition for a rabies vaccine, wherein the immunogenic composition is a protein-form immunogenic composition or a DNA-form immunogenic composition, the DNA is a gene encoding the protein, wherein
the protein-form immunogenic composition comprises:
A. rabies virus structural protein glycoprotein G as a humoral immunogen for activating neutralizing antibodies; and
B. polymerase large protein L as a T-cell immunogen for inducing a cellular immune response;
wherein the DNA-form immunogenic composition comprises:
a. a nucleic acid molecule a encoding the rabies virus structural protein glycoprotein G of item A; and
b. a nucleic acid molecule b encoding the polymerase large protein L of item B.

2. The immunogenic composition for the rabies vaccine according to claim 1, wherein
the rabies virus structural protein glycoprotein G is a full-length amino acid sequence of glycoprotein G, an extracellular domain amino acid sequence of glycoprotein G, or an antigenic fragment of glycoprotein G, wherein
the full-length amino acid sequence of glycoprotein G is selected from the group consisting of:
(1) a polypeptide having the amino acid sequence as shown in SEQ ID NO: 1; and
(2) a polypeptide having at least 80%, at least 85%, at least 90%, at least 91%, at least 92%, at least 93%, at least 94%, at least 95%, at least 96%, at least 97%, at least 98%, or at least 99% homology with the amino acid sequence as shown in SEQ ID NO: 1 and having the same function as SEQ ID NO: 1, the function referring to a function of inducing a humoral immune response and producing the neutralizing antibodies;
the extracellular domain amino acid sequence of glycoprotein G is selected from the group consisting of:
(3) a polypeptide having the amino acid sequence as shown in SEQ ID NO: 2; and
(4) a polypeptide having at least 80%, at least 85%, at least 90%, at least 91%, at least 92%, at least 93%, at least 94%, at least 95%, at least 96%, at least 97%, at least 98%, or at least 99% homology with the amino acid sequence as shown in SEQ ID NO: 2 and having the same function as SEQ ID NO: 1, the function referring to the function of inducing the humoral immune response and producing the neutralizing antibodies; and
the polymerase large protein L is selected from the group consisting of:
(5) a polypeptide having the amino acid sequence as shown in SEQ ID NO: 5; and
(6) a polypeptide having at least 80%, at least 85%, at least 90%, at least 91%, at least 92%, at least 93%, at least 94%, at least 95%, at least 96%, at least 97%, at least 98%, or at least 99% homology with the amino acid sequence as shown in SEQ ID NO: 5 and having the same function as SEQ ID NO: 5, the function referring to a function of inducing the cellular immune response.

3. The immunogenic composition for the rabies vaccine according to claim 2, wherein
when the nucleic acid molecule a encodes the polypeptide of SEQ ID NO: 1, the nucleic acid molecule a is selected from the group consisting of:
(7) the nucleotide sequence as shown in SEQ ID NO: 3; and
(8) a nucleotide sequence having at least 80%, at least 85%, at least 90%, at least 91%, at least 92%, at least 93%, at least 94%, at least 95%, at least 96%, at least 97%, at least 98%, or at least 99% homology with SEQ ID NO: 3;
when the nucleic acid molecule a encodes the polypeptide of SEQ ID NO: 2, the nucleic acid molecule a is selected from the group consisting of:
(9) the nucleotide sequence as shown in SEQ ID NO: 4; and
(10) a nucleotide sequence having at least 80%, at least 85%, at least 90%, at least 91%, at least 92%, at least 93%, at least 94%, at least 95%, at least 96%, at least 97%, at least 98%, or at least 99% homology with SEQ ID NO: 4;
when the nucleic acid molecule b encodes the polypeptide of SEQ ID NO: 5, the nucleic acid molecule b is selected from the group consisting of:
(11) the nucleotide sequence as shown in SEQ ID NO: 6; and
(12) a nucleotide sequence having at least 80%, at least 85%, at least 90%, at least 91%, at least 92%, at least 93%, at least 94%, at least 95%, at least 96%, at least 97%, at least 98%, or at least 99% homology with SEQ ID NO: 6.

4. A use of the immunogenic composition for the rabies vaccine according to any one of claims 1 to 3 in preparation of a rabies vaccine.

5. The use according to claim 4, wherein the nucleic acid molecule a and the nucleic acid molecule b according to claim 1 are inserted into an expression vector to construct a vector vaccine, and the vector used is selected from the group consisting of: nucleic acid vaccine vectors, namely mRNA vectors and DNA plasmid vectors; recombinant viral vectors; and recombinant bacterial vectors.

6. A rabies vaccine capable of simultaneously inducing a humoral immune response and a cellular immune response, obtained by the use according to claim 5, wherein the rabies vaccine is a nucleic acid vaccine, an adenovirus vector vaccine, or an inactivated cell vaccine.

7. The rabies vaccine according to claim 6, wherein the rabies vaccine is an mRNA vaccine constructed by inserting the nucleic acid molecule a and the nucleic acid molecule b according to claim 1 into an mRNA vector, wherein an inserted mRNA expression vector comprises, in order from 5' end to 3' end, a T7 promoter region, a 5' untranslated region, a coding sequence, a 3' non-coding region, a poly(A) tail sequence, and a single restriction enzyme site; wherein the coding sequence is an immunogen coding region comprising, in an N-terminal to C-terminal direction, a first protein sequence, an internal ribosome entry site (IRES) sequence, a second protein sequence, and a degradation domain; wherein the first protein sequence is one of a rabies virus structural protein glycoprotein G coding sequence and a polymerase large protein L coding sequence, and the second protein sequence is the other one of the rabies virus structural protein glycoprotein G coding sequence and the polymerase large protein L coding sequence.

8. The rabies vaccine according to claim 7, wherein the rabies vaccine is a dual-antigen mRNA rabies vaccine, wherein a sequence comprised in the mRNA expression vector is in the following order from 5' end to 3' end: a T7 promoter, a UTR sequence, a first protein domain G protein, an IRES sequence, a second protein domain recombinant L protein, a degradation domain, a 3'-UTR sequence, a poly(A) tail sequence, and a single restriction enzyme site.

9. The rabies vaccine according to claim 8, wherein the rabies vaccine is the dual-antigen mRNA rabies vaccine and a chemically modified mRNA vaccine, wherein the modification comprises replacing at least 50%, at least 60%, at least 70%, at least 80%, at least 90%, or 100% of uracil in an mRNA vaccine backbone with a substance selected from the group consisting of: uridine, N1-methylpseudouridine, N1-ethylpseudouridine, 2-thiouridine, 4'-thiouridine, 5-methylcytosine, 5-methyluridine, 2-thio-1-methyl-1-deazapseudouridine, 2-thio-T-methylpseudouridine, 2-thio-5-azauridine, 2-thiodihydropseudouridine, 2-thiodihydrouridine, 2-thiopseudouridine, 4-methoxy-2-thiopseudouridine, 4-methoxypseudouridine, 4-thio-1-methylpseudouridine, 4-thiopseudouridine, 5-azauridine, dihydropseudouridine, 5-methoxyuridine, and 2'-O-methyluridine.

10. The rabies vaccine according to any one of claims 6 to 9, in addition to an immunogen main body, further comprising one or more pharmaceutically or veterinarily acceptable carriers or excipients, wherein the carriers or excipients are preferably carriers or excipients that render the rabies vaccine suitable for oral, intradermal, subcutaneous, intramuscular, or intranasal administration.
